# EUROPEAN PATENT SPECIFICATION

(11) **EP 1 508 344 B1**
(45) Date of publication and mention of the grant of the patent: **25.10.2006**
(21) Application number: 04019723.8
(22) Date of filing: 19.08.2004
(51) Int. Cl.: A61L 31/04

(54) **Postsurgical adhesion barrier of carboxymethylchitosan and carboxymethylcellulose and method for preparation thereof**
Postchirurgische Antiadhäsionsschicht bestehend aus Carboxymethylchitosan und Carboxymethylcellulose und ihr Herstellungsverfahren
Barrière anti-adhésive postchirurgicale à base de carboxyméthylchitosane et de carboxyméthylcellulose et son procédé de fabrication

(30) Priority: 19.08.2003 CN 03153650
(43) Date of publication of application: 23.02.2005
(73) Proprietor: Dalian Yongxing Medical Material, Co. Ltd., Dalian 116023, Liaoning Province (CN)
(72) Inventor: Zhang, Lianzhong, c/o Dalian Yongxing Med.Material, Dalian 116023, Liaoning Province (CN); Wang, Shuqin, c/o Dalian Yongxing Medical Material, Dalian 116023, Liaoning Province (CN); Feng, Bozhi, c/o Dalian Yongxing Medical Material, Dalian 116023, Liaoning Province (CN)
(74) Representative: Grünecker, Kinkeldey, Stockmair & Schwanhäusser Anwaltssozietät

(56) References cited:
- EP-A- 1 228 771
- WO-A-00/59516
- WO-A-01/05370
- US-A- 5 958 443
- DIAMOND MICHAEL P ET AL: "Reduction of adhesions after uterine myomectomy by Seprafilm membrane (HAL-F): A blinded, prospective, randomized, multicenter clinical study" FERTILITY AND STERILITY, vol. 66, no. 6, 1996, pages 904-910, XP009041574 ISSN: 0015-0282

## Description

### Field of the Invention

The present invention relates to a postsurgical adhesion barrier of carboxymethylchitosan/carboxymethylcellulose cross-linked with multivalent ion and a method for preparation thereof. It belongs to the field of medical material science.

### Background of the Invention

Postsurgical adhesion occurs after virtually all types of surgery and the rate is as high as 60%-90%, resulting in abdominal ache or even bowel obstruction after abdominal operation, pelvic pain or infertility after gynecologic pelvic surgery and significant sequelae after cardiac, cerebral and tendinous surgery. Sometimes, a second operation may be necessary to deal with these postsurgical complications. However, postsurgical adhesion would recur after being eliminated by adhesiolysis. Therefore the presence of postsurgical adhesion is one of the reasons that causes morbidity and mortality of the patients, and lowers the efficacy of medical procedure and imposes a considerable financial burden on the health-care system.

The main reason of postsurgical adhesion formation is that surgery leads to tissue inflammation. The inflammation weakens the function of fibrinolysis in the injured tissue. Therefore, the original balance between fibrinogen deposition and fibrin degradation slants to deposition, and consequently, the possibility that adjacent tissues are connected by coagulated fibrin is increased. At the same time, the inflitrated extracellular matrix (ECM) of surgery could stain the surface of injured tissues, and the adhesion protein in the matrix could adhere to the surface of tissue. Binding to the adhesion protein, the fibroblasts are activated and proliferated on the surface of the wound tissue. The adhesion, activation and proliferation of fibroblasts on the connective tissue lead to postsurgical adhesion.

Postsurgical adhesion has long drawn great attention in the medical field. Doctors tried to prevent or reduce adhesion formation by improving the operation procedure and using various medicaments, such as anti-inflammatory drugs, anticoagulants and fibrinolytic agents. However, the results have not been very encouraging. And the application of those improvements was limited by their side-effects.

In recent years, a new approach, i.e. the applications of physical barrier, including solid membrane and hydrogel, was found to be promising in solving this problem. Aqueous solution of sodium carboxymethylcellulose has been used and produced certain effect (Korean Thorac Cardiovasc Surg 2000; 33:541). Aqueous solution of sodium hyaluronic acid has also been used to prevent postsurgical adhesion (J Invest Surg 1989; 2:320). But the effects are not certain and the price is high (Fertil Steril 1991; 56:563). Hydrogel carboxymethylchitosan has been proved to be effective in reducing postsurgical adhesion by rabbit models (Surg 1996; 120:866 & J Invest surg 2001; 14:93). However, the effect may not be remarkable because it is liquid and easy to be diluted by body fluid and can not stay long at the application site. Expanded polytetrafluoroethylene membrane (Gore-Tex) is the first man-made solid barrier used for preventing postsurgical adhesion. The membrane is confirmed to be effective in pelvic operations (Gynecol Oncol 1993; 48:247 & Prog Clin Biol Res 1993; 381:253). However, it needs to be removed by a second surgical procedure because it is not biodegradable. In addition, the fact that Gore-Tex needs to be sutured to the traumatized surface (surface of the wound) limits its application. Postsurgical adhesion barrier of oxidized regenerated cellulose can be used to reduce pelvic postsurgical adhesion. The problem of the barrier is that it would be ineffective without complete homeostasis since the membrane would become black, brittle and useless when it contacts blood. A number of clinical studies proved the efficacy of another solid barrier, carbodiimide-cross-linked complex of carboxymethylcellulose and sodium hyaluronic acid (Fertil Steril 1996; 66:904). However, the membrane uses the expensive sodium hyaluronic acid as major raw material and its production involves relatively complicated reaction of covalent cross linking which accordingly increases its production cost and sale price. Although the serial materials of polymers of lactic acid and polycaprolactone are biocompatible and biodegradable, they are difficult to be used as postsurgical adhesion barrier because of the inflexibility.

WO 01/05370 discloses an anti-adhesion barrier containing alginate and carboxymethylcellulose.

### Summary of the Invention

The object of the present invention is to provide a biodegradable postsurgical adhesion barrier of the complex of carboxymethylchitosan and carboxymethylcellulose cross-linked with multivalent ions and a method for preparation thereof.

This postsurgical adhesion barrier is a novel medical material. Its specific chemical components include the complex of carboxymethylchitosan and carboxymethylcellulose cross-linked with calcium ion, ferric ion or the mixture of calcium ion and ferric ion and mixture of calcium ion and aluminum ion. Specifically, the multivalent cross-linking ions are divalent calcium ion, trivalent ferric ion or mixed ions of divalent calcium and trivalent ferric and mixed ions of divalent calcium and trivalent aluminum.

The characteristics of the postsurgical adhesion barrier are biocompatible and biodegradable. The postsurgical adhesion barrier of carboxymethylchitosan and carboxymethylcellulose has excellent flexibility, adjustable retention time *in vivo,* simplicity of preparation, significant function of postsurgical adhesion prevention and certain effect of helping wound healing.

The method for the preparation of postsurgical adhesion barrier of carboxymethylchitosan and carboxymethylcellulose is as follows:
Note: In the following descriptions of barrier preparation and the examples, the percentage concentrations of the aqueous solution of ethanol (prepared by adding distilled water to commercially available analytical grade anhydrous ethanol or 95% ethanol) are all in volume : volume(v/v) ratios, that is, the number of milliliters of ethanol in a 100 milliliters of the ethanol aqueous solution. The percentage concentrations of the aqueous solution of carboxymethylchitosan, carboxymethylcellulose, CaCl₂, FeCl₃·6H₂O and AlCl₃·6H₂O are all in weight: volume (w/v) ratios, that is, the number of grams of corresponding matters in a 100 milliliters of respective solution. The concentrations of hydrochloric acid solution and sodium hydroxide solution are presented in equivalent concentration N, that is, the number of gram equivalent of HCl or NaOH dissolved in 1000 milliliters of water.

1. Purification of carboxymethylchitosan and carboxymethylcellulose:
   Medical material is now categorized as medical device in China. Thus the criteria adopted for physicochemical and biological parameters should refer to the medical industry. The contents of heavy metal in carboxymethylchitosan and carboxymethylcellulose should be 10mg/kg (10ppm) or lower. However, the heavy metal contents of certain commercially available carboxymethylchitosan are more than 10 mg/kg (measured by colorimetric method according to Pharmacopoeia of People's Republic of China (2000 Edition)). The unqualified materials can be purified by using the following methods:
   Concentrated hydrochloric acid and purified water such as distilled water, double distilled water or medical pure water were used to prepare 0.5-2 N hydrochloric acid solution. Anhydrous ethanol or 95% ethanol was added to the hydrochloric acid solution to produce a mixed solution with the volume-to-volume ratio of ethanol and hydrochloric acid solution ranging from 6:4 to 8:2. The carboxymethylchitosan to be purified was soaked in the mixed solution for 15~180 minutes, and then isolated by filtration. The carboxymethylchitosan was washed with 50~75% ethanol aqueous solution and the ethanol solution was discarded by filtration. The carboxymethylchitosan is then maintained in a 70%~80% ethanol aqueous solution, 1~2N NaOH aqueous solution was added drop wise to adjust the pH value to 7-8. The purified carboxymethylchitosan was isolated by filtration, washed again with 70~80% ethanol aqueous solution and dried at room temperature, in vacuum or under flowing air.
   Carboxymethylcellulose can be obtained from pure viscose fiber by carboxyl methylation (Chinese Patent No. 001 03506.1) or commercially available product. If the heavy metal content is more than 10 mg/kg(10ppm), it can be purified by the same procedure used for the purification of carboxymethylchitosan.
   Those raw materials of carboxymethxylchitosan and carboxymethylcellulose, which meet the criteria of heavy metal content and other criteria of membrane preparation can be used directly.
2. The 1%~5% carboxymethxylchitosan solution and 2%~5%
   Carboxymethylcellulose solution were prepared by dissolving carboxymethxylchitosan with degree of substitution of 0.6-1.2, heavy mental content equal to or less than 10 mg/kg, and carboxymethylcellulose with degree of substitution of 0.4-1.2 and heavy mental content equal to or less than 10 mg/kg in purified water, respectively, at temperature ranging between 25~50°C with stirring. The solution was left standing for enough time in order to obtain even solution, and then filtered to remove precipitates.
3. Certain amount of the prepared carboxymethxylchitosan aqueous solution, or mixed solution of carboxymethxylchitosan and carboxymethylcellulose with the weight ratio of carboxymethxylchitosan and carboxymethylcellulose from 1:0.05 to 1:2 was applied to clean plates, such as clean metal (e.g. stainless steel) plate, glass plate or resin plate and the like. The plates were dried with the application at 25~50°C in a vacuum dryer or under flowing air. The thickness of the dried membrane was controlled to 20~60µm by adjusting the concentration (C) of carboxymethxylchitosan and carboxymethylcellulose, and the volumes of the carboxymethxylchitosan solution or the mixed solution of carboxymethxylchitosan and carboxymethylcellulose applied. In other words, the thickness of the dried membrane was controlled by the substantial mass of carboxymethxylchitosan and carboxymethylcellulose applied (M=CV, such as M=2-4 mg/cm²).
4. 1.5%~15% CaCl₂, 2%~15% FeCl₃·6H₂O and 1%~15% AlCl₃·6H₂O were prepared with pure water, respectively. A mixed solution of CaCl₂/FeCl₃ with Ca⁺⁺ to Fe⁺⁺⁺ ion equivalent ratios from 1 :10 to 10:1 was prepared using the above CaCl₂ and FeCl₃ aqueous solutions. And a mixed solution of CaCl₂/AlCl₃ with the Ca⁺⁺ to Al⁺⁺⁺ ion equivalent ratios from 1:1 to 20:1 was prepared using the above CaCl₂ and AlCl₃ aqueous solutions.
5. The dried membrane of the mixture of carboxymethxylchitosan and carboxymethylcellulose prepared in Step 3 was soaked in any of the solutions of CaCl₂, FeCl₃, mixture of CaCl₂ and FeCl₃ or mixture of CaCl₂ and AlCl₃ for 15~180 minutes at room temperature, sufficiently washed with distilled water, and dried in the shade to obtain water insoluble postsurgical adhesion barrier of the complex of carboxymethylchitosan and carboxymethylcellulose cross-linked with calcium ion, ferric ion, mixed ions of calcium and ferric or mixed ions of calcium and aluminum.
6. The cross-linked postsurgical adhesion barrier of the complex of carboxymethylchitosan and carboxymethylcellulose was then sealed by radiation-resistant materials, such as aluminum-plastic composite foil, PETG polyester film or high density polyethylene film, and sterilize by ⁶⁰Co γ ray irradiation.

The postsurgical adhesion barrier of carboxymethylchitosan or the complex of carboxymethylchitosan and carboxymethylcellulose thus prepared can be used to prevent postsurgical adhesion when attached to the wound before suturing of incision.

The chemical structure and mechanism of the postsurgical adhesion barrier of carboxymethylchitosan and carboxymethylcellulose of the invention are as follows:

Carboxymethylchitosan and carboxymethylcellulose, which are the major components of the barriers, are the derivatives of chitin and cellulose. Chitin and cellulose exist most commonly as natural polysaccharides.

The raw materials for the preparation of carboxymethylchitosan are the crusts of shrimps and crabs. Chitin is extracted from the crusts. The structure of chitin is poly-N-acetyl-glucosamine-[β-(1,4)-2-acetylamine-2-deoxy-D-glucose]. Chitin can be converted to poly-glucosamine, that is, chitosan, by deacetylation under alkaline conditions. Carboxymethylchitosan is prepared by alkalinization and then carboxymethylation of chitosan using chloroacetic acid as major reacting agent. Carboxymethylchitosan can also be produced in batch in the industry. However, the quality is not stable. Carboxymethylchitosan is naturally a polymer ampholine, because carboxymethyl moiety (CH₂-COO⁻) is anion and carboxymethylated amido and protonated un-carboxymethylated amido are cation. Carboxymethylchitosan, like its precursor chitosan and chitin, has excellent biocompatibility. Its degradation product glucosamine is bioresorbable.

Natural cellulose as the raw material for carboxymethylcellulose is used. The structure of cellulose is poly-glucose-[β-(1,4)-2-deoxy-D-glucose] and converted to carboxymethylcellulose by carboxymethylation. Carboxymethylcellulose also can be produced in batch in the industry. It is an electronegative polymer electrolyte because the carboxymethyl moiety (CH₂COO⁻) is anion. And it has good biocompatibility and bioresorbability since it is an improved polysaccharide and its low molecular weight degradation product glucose is able to be bio-metabolized.

It has been proved that carboxymethylchitosan can prevent postsurgical adhesion by a rabbit animal model (Surg 1996; 120:866 & J Invest surg 2001; 14:93). However, the effect is not satisfactory since it is liquid *in vivo* and easily diluted by body fluid thus quickly disappears from the site of application. The carboxymethyl moiety (CH₂-COO⁻) of carboxymethylchitosan can react with multivalent cation to form a cross-linked water insoluble polysaccharide. This invention produces a water insoluble film of carboxymethylchitosan cross-linked with multivalent cations, such as Ca⁺⁺, Fe⁺⁺⁺, Ca⁺⁺/Fe⁺⁺⁺ or Ca⁺⁺/Al⁺⁺⁺, which has longer retention time in body to completely isolate the traumatic tissues in the wound healing period, hence improves the function of postsurgical adhesion prevention. Carboxymethylchitosan film becomes hydrogel and solution again during the period of wound healing and post wound healing owing to the ion exchange reactions of the multivalent ions with Na⁺ and K⁺ in the body fluid which substitute the multivalent metal ions in the membrane. The hydrogel and solution of carboxymethxylchitosan thus formed viscous solution barrier further exert functions of postsurgical adhesion prevention. Then the hydrogel carboxymethylchitosan will be gradually degraded into oligose solution, further turns into low molecular saccharides and finally enter normal metabolism. The disadvantage of the cross-linked carboxymethylchitosan film is fragility. The film of the Ca⁺⁺ cross-linked carboxymethylchitosan is a little better. Attention should be paid to the drying speed and moisture content of the membrane to avoid cracking. The disadvantage of the membrane of cross-linked carboxymethylchitosan can be overcome by adding carboxymethylcellulose component to produce a complex membrane of carboxymethylchitosan and carboxymethylcellulose. In the complex membrane of carboxymethylchitosan and carboxymethylcellulose, all the -CH₂-COO⁻ groups in both of the polysaccharides are electrostatically attracted to amino group of carboxymethylchitosan, forming a complex amphiprotic polymer electrolyte. The complex is also water soluble. The anti-adhesion effect of the complex may not be satisfactory since it will be diluted by body fluid and removed quickly from the wound as in the case of carboxymethxylchitosan. The insoluble film is prepared by cross-linking the complex of carboxymethylchitosan and carboxymethylcellulose with Ca⁺⁺, Fe⁺⁺⁺, Ca⁺⁺/Fe⁺⁺⁺ or Ca⁺⁺/Al⁺⁺⁺. For the same reason, the retention time in the body of the complex film becomes longer and its anti-adhesion effect is improved. The polysaccharide complex would turn into hydrogel and solution during the periods of wound healing and post wound healing through ion exchanging reactions, that is, the substitution of the multivalent ions in the complex with the monovalent ions in body fluid, such as Na⁺ and K⁺. Then it is further biodegraded to oligosaccharide and undergoes normal metabolism *in vivo.* The toughness can be adjusted by the amount of carboxymethylcellulose added. That is, higher percentage of carboxymethylcellulose makes the membrane tougher. Ions exchanging rate *in vivo* can be adjusted through adjusting the ion species and ratio of mixed ions. For example, the exchanging rate of the calcium ions in the calcium ion cross-linked membrane with sodium ion in body fluid is much higher than that of sodium ion in the body fluid with Fe⁺⁺⁺ ion, Al⁺⁺⁺ ion or mixed ions in the membrane cross-linked by Fe⁺⁺⁺ or mixed ions of Ca⁺⁺/Fe⁺⁺⁺ and Ca⁺⁺/Al⁺⁺⁺.

Consequently, the retention time of the cross-linked membrane *in vivo* can be adjusted as mentioned above in order to produce barriers suitable to different kinds of surgeries.

The animal experiments showed that a better anti-adhesion effect could be obtained by firstly coating the wound tissue with solutions of carboxymethylchitosan and carboxymethylcellulose before applying the membrane. This approach also makes it easier for the membrane attaching to the traumatized surface. For this reason, the carboxymethylchitosan/carboxymethylcellulose solution should be sterilized, by being filtrated through 0.2 µm millipore membrane and sealed under asepsis condition.

### Detailed Description of the Invention:

### Example 1

(1) A commercially available powder of carboxymethylchitosan with Degree of substitution equal to 1.0 was used as raw material. Its heavy metal content measured by colorimetric method according to "Pharmacopoeia of People's Republic of China" (2000 Edition) was found to be higher than 10 mg/kg (10 ppm). Therefore it was purified according to the following procedure:
   A 1 N hydrochloric acid solution was prepared with concentrated hydrochloric acid and double distilled water, then 150 ml ethanol/HCl solution mixture with volume-to-volume ratio of 7:3 was prepared by adding anhydrous ethanol to the 1 N HCl solution. A 25 g carboxymethylchitosan powder was soaked in the above solution for 30 min at room temperature, then transferred to a vacuum glass filter and filtered under negative pressure produced by water circulating pump. The powder was washed 5 times with 5 x 150 ml 50% ethanol solution, filtered under negative pressure, and transferred to a beaker. A 150 ml 75% ethanol aqueous solution was added to the beaker and the solid liquid mixture was stirred to produce a suspension. A 2 N NaOH aqueous solution was added drop wise to the slurry with stirring while the pH value of the suspension was monitored with a pH meter until the pH value was stable at 7.4. Then the slurry was transferred to a vacuum glass filter and filtered under negative pressure. The treated carboxymethylchitosan was washed 3 times with 3 x 150 ml 75% ethanol aqueous solution and dried in a vacuum dryer at room temperature and. 18 g purified product was obtained. The heavy metal content of the product was found to be less than 10 mg/kg (10 ppm) measured by colorimetric method according to "Pharmacopoeia of People's Republic of China" (2000 Edition).
(2) The purified carboxymethylchitosan was dissolved with double distilled water, at 40°C with electromagnetic stirring to produce a 2.5% aqueous solution which was allowed to stand overnight. Then the solution was filtered and the filtrate was degassed.
(3) 3x 10 ml of above solutions were applied to 3 clean PETG polyester plates (7 cm x 9 cm), dried under clean flowing air at room temperature to obtain plate attached films.
(4) 400 ml of 5% CaCl₂ (analytical pure grade) solution, 100 ml of 8.1% FeCl₃·6H₂O (analytical pure grade) solution and 100 ml of AlCl₃·6H₂O (analytical pure grade) solution were prepared with double distilled water. The equivalent concentration of ions of the above three solutions are all 0.9. A mixed solution of 100 ml CaCl₂ and 100 ml FeCl₃ with ion equivalent ratio of 1:1 for Ca⁺⁺ : Fe⁺⁺⁺ and another mixed solution of 100 ml CaCl₂ and 100 ml AlCl₃ with ion equivalent ratio of 1:1 for Ca⁺⁺: Al⁺⁺⁺ were prepared with the three solutions prepared above. Three PETG polyester plates with carboxymethylchitosan films attached were soaked in CaCl₂ solution, mixed solution of CaCl₂ and FeCl₃ and mixed solution of CaCl₂ and AlCl₃ for 150 min. Then those plates were taken out and washed 3 times with distilled water, dried in the shade at room temperature and three water insoluble carboxymethylchitosan films with a thickness of 38 µm were detached from the plates.
(5) The above carboxymethylchitosan films were sealed in aluminum-plastic foil bags and sterilized by ⁶⁰Co irradiation and the postsurgical adhesion barriers were produced. The ion exchange rate of calcium ion in the barriers with sodium ion in saline was found to be high. Therefore the barriers cross-linked with calcium ion are suitable to surgeries with short healing period. On the other hand, the membrane cross linked with mixed ions of calcium and ferric, and mixed ions of calcium and aluminum, are suitable to surgeries with longer healing periods.

### Example 2:

(1) A commercially available powder of carboxymethylchitosan with Degree of substitution equal to 1.0 was used as raw material. Its heavy metal content measured by colorimetric method according to "Pharmacopoeia of People's Republic of China" (2000 Edition) was found to be higher than 10 mg/kg (10 ppm). Therefore it was purified according to the following procedure:
   An 1 N hydrochloric acid solution was prepared with concentrated hydrochloric acid and double distilled water, then 120 ml ethanol/HCl solution with volume-to-volume ratio of 7:3 was prepared by adding anhydrous ethanol to the 1 N HCl solution.. A 20 g carboxymethylchitosan powder was soaked in the above solution for 20 min at room temperature, then transferred to a vacuum glass filter and filtered under negative pressure produced by water circulating pump. The powder was washed 5 times with 5 x 120 ml 50% ethanol solution, filtered under negative pressure, and transferred to a beaker. A 120 ml 75% ethanol aqueous solution was added to the beaker and the solid liquid mixture was stirred to produce a suspension. A 2 N NaOH aqueous solution was added dropwise to the slurry with stirring while the pH value of the suspension was monitored with a pH meter until the pH value was stable at 7.6. Then the slurry was transferred to a vacuum glass filter and filtered under negative pressure. The treated carboxymethylchitosan was washed 3 times with 3 x 120 ml 75% ethanol aqueous solution and dried in a vacuum dryer at room temperature. 15 g purified product was obtained. The heavy metal content of the product was found to be less than 10 mg/kg (10 ppm) measured by colorimetric method according to "Pharmacopoeia of People's Republic of China" (2000 Edition).
   Carboxymethylcellulose with degree of substitution equal to 0.59 was prepared by carboxymethylation of non-woven viscose fiber fabric. The heavy metal content was less than 10 mg/kg (10 ppm) measured by colorimetric method according to "Pharmacopoeia of People's Republic of China" (2000 Edition).
(2) The above purified carboxymethylchitosan and the carboxymethylcellulose were separately dissolved in double distilled water at 40°C with electromagnetic stirring to prepare 2.5% aqueous solutions of the two polysaccharides,. The solutions were left standing overnight. Then the solution was filtered and the filtrate was collected. Eight mixed solutions of carboxymethylchitosan and carboxymethylcellulose were prepared with mass ratios of 1:2, 1:1, 1:0.3, 1:0.2, 1;0.15, 1:0.11, 1:0.07 and 1:0.05 of the two polymers and degassed.
(3) 10 ml of each of the mixed solutions prepared above were applied to 8 clean stainless steel plates (7 cm x 9 cm) and dried in a vacuum dryer at 45°C to obtain plate attached films.
(4) A 500 ml solution of 5% CaCl₂ (analytical pure grade) was prepared with double distilled water in an enamel dish. The stainless steel plates with films attached were soaked in CaCl₂ solution for 120 min. Then those plates were taken out and washed 3 times with distilled water, dried in the shade at room temperature and eight water insoluble films of the complex of carboxymethylchitosan and carboxymethylcellulose cross-linked with calcium ion and with thickness of 38µm were detached from the plates. The toughness and flexibility of the film increased and the transparency of the film decreased along with the increase of carboxymethylcellulose component in the films.
(5) The above films of carboxymethylchitosan and carboxymethylcellulose were sealed in aluminum-plastic foil bags and sterilized by ⁶⁰Co irradiation and the postsurgical adhesion barriers were produced. The ion exchange rate of calcium ion in the barriers with sodium ion in physiological saline was found to be high. Therefore, the barriers cross-linked with calcium ion are suitable to surgeries with short healing period.

### Example 3

(1) A commercially available powder of carboxymethylchitosan with degree of substitution equal to 0.8 was used as raw material. Its heavy metal content measured by colorimetric method according to "Pharmacopoeia of People's Republic of China" (2000 Edition) was found to be higher than 10 mg/kg (10 ppm). Therefore it was purified according to the following procedure:
   A 1 N hydrochloric acid solution was prepared with concentrated hydrochloric acid and double distilled water, then 200 ml ethanol/HCl solution with volume-to-volume ratio of 7:3 was prepared by adding anhydrous ethanol to the 1 N HCl solution. A 33 g carboxymethylchitosan powder was soaked in the above solution for 40 min at room temperature, then transferred to a vacuum glass filter and filtered under negative pressure produced by water circulating pump. The powder was washed 5 times with 5 x 180 ml 50% ethanol aqueous solution, filtered under negative pressure, and transferred to a beaker. A 200 ml 75% ethanol aqueous solution was added to the beaker and the solid liquid mixture was stirred to produce a suspension. 2 N NaOH aqueous solution was added drop wise to the slurry with stirring while the pH value of the suspension was monitored with an pH meter until the pH value was stable at 7.5. Then the slurry was transferred to a vacuum glass filter and filtered under negative pressure. The treated carboxymethylchitosan was washed 3 times with 3 x 180 ml 75% ethanol aqueous solution and dried under vacuum in a vacuum dryer at room temperature. 25 g purified product was obtained. The heavy metal content of the product was found to be less than 10 mg/kg (10 ppm) measured by colorimetric method according to "Pharmacopoeia of People's Republic of China" (2000 Edition).
   Carboxymethylcellulose with degree of substitution equal to 0.61 was prepared by carboxymethylation of non-woven viscose fiber fabric. The heavy metal content was less than 10 mg/kg (10 ppm) measured by colorimetric method according to "Pharmacopoeia of People's Republic of China" (2000 Edition).
(2) The above purified carboxymethylchitosan and the carboxymethylcellulose were separately dissolved in double distilled water at 40°C with electromagnetic stirring to prepare 2.5% aqueous solutions of the two polysaccharides. The solutions were left standing overnight. Then the solutions were filtered and the filtrates were collected. A mixed solution with a mass ratio of 1:0.2 of carboxymethylchitosan and carboxymethylcellulose was prepared and degassed.
(3) 10 ml of the above solution was applied to a clean PETG polyester plates (7 cm x 9 cm), dried in a vacuum dryer at 45°C to obtain plate attached films.
(4) A 200 ml solution of 8.1% FeCl₃·6H₂O (analytical pure grade) was prepared with double distilled water in an enamel dish. The PETG polyester plate with film of mixed carboxymethylchitosan and carboxymethylcellulose attached was soaked in FeCl₃ aqueous solution for 60 min. Then this plate was washed 3 times with distilled water, dried in the shade at room temperature and an insoluble membrane of the complex of carboxymethylchitosan and carboxymethylcellulose cross-linked with Fe⁺⁺⁺ and with thickness of 37 µm was detached from the plate.
(5) The above films was sealed in aluminum-plastic foil bags and sterilized by ⁶⁰Co irradiation. The ion exchange rate of ferric ion in the barriers with sodium ion in physiological saline was found to be slow. Therefore, the barriers cross-linked with Fe⁺⁺⁺ are suitable for surgeries with a longer healing period.

### Example 4

(1) A commercially available powder of carboxymethylchitosan with Degree of substitution equal to 0.8 was used as raw material. Its heavy metal content measured by colorimetric method according to "Pharmacopoeia of People's Republic of China" (2000 Edition) was found to be higher than 10 mg/kg (10 ppm) . Therefore it was purified according to the following procedure:
   A 1 N hydrochloric acid solution was prepared with concentrated hydrochloric acid and double distilled water, then 100 ml ethanol/HCl solutions with volume-to-volume ratio of 7:3 was prepared by adding anhydrous ethanol to the 1 N HCl solution. A 15 g carboxymethylchitosan powder was soaked in the above solution for 30 min at room temperature, then transferred to a vacuum glass filter and filtered under negative pressure produced by water circulating pump. The powder was washed 5 times with 5 x 100 ml 50% ethanol aqueous solution, filtered under negative pressure, and transferred to a beaker. A 100 ml 75% ethanol aqueous solution was added to the beaker and the solid liquid mixture was stirred to produce a suspension. 2 N NaOH aqueous solution was added drop wise to the slurry with stirring while the pH value of the suspension was monitored with a pH meter until the pH value was stable at 7.6. Then the slurry was transferred to a vacuum glass filter and filtered under negative pressure. The treated carboxymethylchitosan was washed 3 times with 3 x 100 ml 75% ethanol aqueous solution and dried under vacuum in a vacuum dryer at room temperature and. 12 g purified product was obtained. The heavy metal content of the product was found to be less than 10 mg/kg (10 ppm) measured by colorimetric method according to "Pharmacopoeia of People's Republic of China" (2000 Edition).
   Carboxymethylcellulose with degree of substitution equal to 0.48 was prepared by carboxymethylation of non-woven viscose fiber fabric. The heavy metal content was less than 10 mg/kg (10 ppm) measured by colorimetric method according to "Pharmacopoeia of People's Republic of China" (2000 Edition).
(2) The above purified carboxymethylchitosan and the carboxymethylcellulose were separately dissolved in double distilled water at 40°C with electromagnetic stirring to prepare 2.5% aqueous solutions of the two polysaccharides. The solutions were left standing overnight. Then the solutions were filtered and the filtrates were collected. A mixed solution with a mass ratios of 1:1 of carboxymethylchitosan and carboxymethylcellulose was prepared and degassed.
(3) 3x10 ml of the above solution were separately applied to 3 clean glass plates (7 cm x 9 cm), dried in a vacuum dryer at 45°C to obtain plate attached films.
(4) A 300 ml solution of 5% CaCl₂ (analytical pure grade) and 300 ml of 8.1% FeCl₃·6H₂O (analytical pure grade) solution were prepared with double distilled water and the ion equivalent concentrations of both solutions were 0.9. Mixed solutions of 150 ml CaCl₂ and 50 ml FeCl₃, 100 ml CaCl₂ and 100 ml FeCl₃, and 50 ml CaCl₂ and 150 ml FeCl₃ were prepared in three enamel dishes, and the ion equivalent ratios of calcium ion to ferric ion were 1:3, 1:1, 3:1, respectively. The three glass plates with film attached were separately soaked in the three mixed solutions for 90 min. Then those plates were taken out and washed 3 times with distilled water, dried in the shade at room temperature and three water insoluble films of carboxymethylchitosan and carboxymethylcellulose cross-linked with calcium ion and ferric ion and with thickness of 37 µm were detached from the plates. The color of the membrane turned from light yellow to brown as the portion of ferric ion in the mixed ions increased.
(5) The films were then sealed in aluminum-plastic foil bags and sterilized by ⁶⁰Co irradiation. The ion exchange rate of calcium and ferric ions in the membrane with sodium ion in saline decreased as the portions of ferric ions in the mixed ion solution increased, Therefore the barriers cross-linked with calcium and ferric ions are suitable for surgeries with longer healing period.

### Example 5

(1) A commercially available powder of carboxymethylchitosan with degree of substitution equal to 0.6 was used as raw material. The content of heavy metals of the material was found to be less than 10 mg/kg (10 ppm) measured by colorimetric method according to "Pharmacopoeia of People's Republic of China" (2000 Edition).
   Carboxymethylcellulose with degree of substitution equal to 0.63 was prepared by carboxymethylation of non-woven viscose fiber fabric. The heavy metal content was less than 10 mg/kg (10 ppm) measured by colorimetric method according to "Pharmacopoeia of People's Republic of China" (2000 Edition).
(2) The above carboxymethylchitosan and the carboxymethylcellulose were separately dissolved in double distilled water at 40°C with electromagnetic stirring to prepare 2.5% aqueous solutions of the two polysaccharides. The solutions were left standing overnight, filtered and the filtrates were collected. A mixed solution with a mass ratio of 1:0.5 of the two polymers was prepared and degassed.
(3) 5 x 10 ml of the above solutions were separately applied to 5 clean glass plates (7 cm x 9 cm), dried in a vacuum dryer at 45°C to obtain plate attached films.
(4) An 800 ml solution of 5% CaCl₂ (analytical pure grade) and 250 ml of 7.25% AlCl₃·6H₂O (analytical pure grade) solution were prepared with double distilled water and the ion equivalent concentration of both solutions were 0.9. Mixed solutions of 200 ml CaCl₂ and 10 ml AlCl₃ solution, 200 ml CaCl₂ and 20 ml AlCl₃ solution, 150 ml CaCl₂ and 30 ml AlCl₃, 150 ml CaCl₂ and 50 ml AlCl₃ solution, 100 ml CaCl₂ and 100 ml AlCl₃ solution were prepared in five enamel dishes, and the ion equivalent ratios of calcium ion to aluminum ion were 20:1, 10:1, 5:1, 3:1 and 1:1 respectively. The five glass plates with films attached were separately soaked in the five mixed solutions for 90 min. Then those plates were taken out and washed 3 times with distilled water, dried in the shade at room temperature and five water insoluble films of carboxymethylchitosan and carboxymethylcellulose cross-linked with mixed ions of calcium and aluminum and with thickness of 38 µm were detached from the plates. The portion of aluminum ion in the mixed ions did not influence the appearance of the membrane.
(5) The above films of carboxymethylchitosan and carboxymethylcellulose were sealed in aluminum-plastic foil bags and sterilized by ⁶⁰Co irradiation. The ion exchanging rate of calcium and aluminum ions in the membrane with sodium ions in physiological saline decreased as the portion of aluminum ion in the mixed solutions increased, hence the membrane cross-linked with mixed ions of calcium and aluminum is suitable for surgeries with different healing period.

### Example 6: Preventing postsurgical adhesion in rats

The animal experiment of this example was completed in the National Key Laboratory of Natural Medicine and Bionic Medicine at Peking University.
(1) A commercially available powder of carboxymethylchitosan with Degree of substitution equal to 1.0 used as raw material. Its heavy metal content measured by colorimetric method according to "Pharmacopoeia of People's Republic of China" (2000 Edition) was found to be higher than 10 mg/kg (10 ppm). Therefore it was purified according to the following procedure:
   A 1 N hydrochloric acid solution was prepared with concentrated hydrochloric acid and double distilled water, then 150 ml ethanol/HCl solution with volume-to-volume ratio of 7:3 was prepared by adding anhydrous ethanol to the 1 N HCl solution. 25 g carboxymethylchitosan powder was soaked in the above solution for 25 min at room temperature, then transferred to a vacuum glass filter and filtered under negative pressure produced by water circulating pump. The powder was washed 5 times with 5 x 150 ml 50% ethanol aqueous solution, filtered under negative pressure, then transferred to a beaker. A 150 ml 75% ethanol aqueous solution was added to the beaker and the solid liquid mixture was stirred to produce a suspension. 2 N NaOH aqueous solution was added drop wise to the slurry with stirring while the pH value of the suspension was monitored with a pH meter until the pH value was stable at 7.5. Then the slurry was transferred to a vacuum glass filer and filtered under negative pressure. The treated carboxymethylchitosan was washed 3 times with 3 x 150 ml 75% ethanol aqueous solution and dried under vacuum in a vacuum dryer at room temperature and. 19 g purified product was obtained. The heavy metal content of the product was found to be less than 10 mg/kg (10 ppm) measured by colorimetric method according to "Pharmacopoeia of People's Republic of China" (2000 Edition).
   Carboxymethylcellulose with degree of substitution equal to 0.60 was prepared by carboxymethylation of non-woven viscose fiber fabric. The heavy metal content was found to be less than 10 mg/kg (10 ppm) measured by colorimetric method according to "Pharmacopoeia of People's Republic of China" (2000 Edition).
(2) The above purified carboxymethylchitosan and the carboxymethylcellulose were separately dissolved in double distilled water at 40°C with electromagnetic stirring to prepare 2.5% aqueous solutions of the two polysaccharides. The solutions were left standing overnight and filtered and the filtrates were collected. A mixed solution of carboxymethylchitosan and carboxymethylcellulose with a mass ratio of 1:0.05 was prepared and degassed. Half of the mixed solution was used to produce the membrane as outlined in steps (3)-(5). The other half was used to prepare a sterilized mixed solution of carboxymethylchitosan and carboxymethylcellulose as described in Step (6).
(3) 5x10 ml of the above solution were separately applied to 5 clean PETG polyester plates (7 cm x 9 cm), dried in a vacuum dryer at 45°C to obtain plate attached films.
(4) A 500 ml solution of 5% CaCl₂ (analytical pure grade) was prepared with double distilled water in an enamel dish. The PETG polyester plates with films attached were soaked in CaCl₂ solution for 150 min. Then those plates were taken out and washed 3 times with distilled water and dried in the shade at room temperature. Five water insoluble films of carboxymethylchitosan and carboxymethylcellulose cross- linked with calcium ion and with thickness of 38 µm were detached from the plates
(5) The above films were sealed in aluminum-plastic foil bags and sterilized by ⁶⁰Co irradiation.
(6) The second half of the mixed solution of carboxymethylchitosan and carboxymethylcellulose from Step (2) was filtered under negative pressure produced by water circulating pump through 1.2 µ, 1.0 µ, 0.8 µ, 0.6 µ, 0.45 µ millipore membranes sequentially. Then the filtrate was poured into a 10 ml sterilized syringe equipped with 0.2 µ millipore membrane (produced by Sartorius Co.) and filtered under aseptic conditions. The obtained mixed solution of carboxymethylchitosan and carboxymethylcellulose was sealed in a 5 ml sterilized syringe under aseptic condition, and the syringe was sealed using aluminum-plastic foil, which had been sterilized by ⁶⁰Co γ ray irradiation before.
(7) 27 SD rats (180 g-210 g) were assigned to two groups with 16 rats in the control group and 11 rats in the experimental group. Rats were anesthetized with pentobarbital sodium intraperitoneally (50 mg/kg) followed by hair removal from the abdominal area. After the abdomen was exposed through a ventral midline incision, 5 cm ileum, which was 5 cm from the cecum, was exteriorized and abraded with dry gauze until punctuate bleeding occurred. For the control group, the intestine was replaced the abdomen was closed and coated with erythromycin, and the animals were fed 12 hours later. For the experimental group, the injured area of abraded intestine was immediately coated with sterilized solution of carboxymethylchitosan and carboxymethylcellulose prepared in Step (6) and then wrapped with a rectangular postsurgical adhesion barrier prepared in Step (5). Finally, the intestine was replaced, the abdomen was closed and coated with erythromycin, and the animals were fed 12 hours later.

After 14 days, all animals were sacrificed and the abdominal cavities were opened. The adhesion degrees were observed by macromethod and classified into 4 grades refering to the taxonomy of Jianhua Hu (Chinese Journal of Experimental Surgery, 1989, 6(3), 101) and Phillips(Br J Sug, 1984, 71(1), 537):
Grade 0: no adhesion
Grade I: little loosening adhesion to the adjacent tissues (omentum, peritoneum, mesentery); one adhesion band, adhesion isolated easily without bleeding
Grade II: more compact adhesion; two adhesion bands, isolated with little trauma and bleeding
Grade III: compact and comprehensive adhesion; more than two adhesion bands or chordal bands
Grade IV: compact, comprehensive and blocking adhesion; unable to be blunt dissected; existing intestine obstruction

### Statistical analysis:

The results of the control group and the experimental group were statistically analyzed using χ² test. If P<0.05, the results show significant difference between the experimental group and the control.

### Pathological analysis of tissues:

The ileocaecocolica was fixed by 10 % formaldehyde, paraffin embedding and section cutting (5 µm), staining with H.E. The healing condition, numbers and arranging of mesothelial cells, fibroblasts and collagen fibers, tissue inflammation and the like were observed by optical microscope.

Results: The adhesion barriers used in the experimental rats were absorbed completely in two weeks and no residues of the barriers were observed by macromethod. The degrees of adhesion of rats in the two groups were shown in the following table:

| Grade | 0 | I | II | III | IV | Total rats |
|---|---|---|---|---|---|---|
| Control group | 1 | 1 | 0 | 14 | 0 | 16 |
| Experimental group | 3 | 0 | 5 | 3 | 0 | 11 |

The results were statistically analyzed with χ² test and the P value was calculated to be smaller than 0.001, demonstrating that the barrier can significantly reduce or prevent postsurgical adhesion.

The results of pathological analysis of tissues were obtained by using a microscope:
Control group: absence of ciliated membrane mesothelium, interstitial oedema, formation of granulation, active reproduction of fibroblasts and proliferating blood vessel inflammation.
Experimental group: relatively milder reaction of interstitial reaction, thinner fibrosis zone and only local fibrosis.

The results of the tissue pathological analysis show the same effect of the barrier in the prevention of postsurgical adhesion. Furthermore, the results also demonstrate that the barrier has certain effect on wound healing as evidenced by the observations including minor interstitial reactions, absence of granulation and blood vessel inflammation, and inactiveness of fibroblasts in the experimental group.

## Claims

1. A postsurgical adhesion barrier, comprising:
carboxymethylchitosan;
carboxymethylcellulose; and
a cross-linking agent of metal ions.

2. The postsurgical adhesion barrier according to Claim1, wherein the degree of substitution of carboxymethyl moiety of the carboxymethylchitosan is 0.6 to 1.2.

3. The postsurgical adhesion barrier according to Claim 1, wherein the degree of substitution of carboxymethyl moiety of the carboxymethylcellulose is 0.4 to 1.2.

4. The postsurgical adhesion barrier according to Claim 1, wherein the weight ratio of carboxymethxylchitosan and carboxymethylcellulose is from 1:0.05 to 1:2.

5. The postsurgical adhesion barrier according to Claim1, wherein the cross-linking agent is selected from the group consisting of Ca⁺⁺, Fe⁺⁺⁺, a mixture of Ca⁺⁺ and Fe⁺⁺⁺, and a mixture of Ca⁺⁺ and Al⁺⁺⁺.

6. The postsurgical adhesion barrier according to Claim 1 or 5, wherein the metal ions are derived from CaCl₂, FeCl₃·6H₂O and AlCl₃·6H₂O, respectively.

7. The postsurgical adhesion barrier according to Claim 1 or 5, wherein the ion equivalent ratios of Ca⁺⁺ to Fe⁺⁺⁺ of the mixture of Ca⁺⁺ and Fe⁺⁺⁺ is from 1:10 to 1:0.

8. The postsurgical adhesion barrier according to Claim 5, wherein the ion equivalent ratios of Ca⁺⁺ to Al⁺⁺⁺ of the mixture of Ca⁺⁺ and Al⁺⁺⁺ is from 1:1 to 1:0.05.

9. A method for preparing a postsurgical adhesion barrier, comprising the following steps of:
(1) preparing, respectively, a first polymer aqueous solution of carboxymethxylchitosan and a second polymer aqueous solution of carboxymethylcellulose;
(2) preparing an aqueous solution of a metal cross-linking agent;
(3) applying a mixture of the first polymer aqueous solution and the second polymer aqueous solution to the surface of a plate, drying the applied polymer aqueous solution on the plate to obtain a film on the plate;
(4) soaking the dried film on the plate of Step (3) in the aqueous solution of Step (2) to complete cross-linking reaction; and
(5) drying the cross-linked film on the plate of Step (4), then detaching the film from the plate.

10. The method according to Claim 9, wherein the concentration of the carboxymethxylchitosan in the first polymer aqueous solution is 1%to 5% (g/100 ml).

11. The method according to Claim 9, wherein the concentration of the carboxymethylcellulose in the second polymer aqueous solution is 2%to 5% (g/100 ml).

12. The method according to Claim 9, wherein the said metal cross-linking agent is selected from a group consisting of Ca⁺⁺, Fe⁺⁺⁺, a mixture of Ca⁺⁺ and Fe⁺⁺⁺, and a mixture of Ca⁺⁺ and Al⁺⁺⁺.

13. The method according to Claim 12, wherein the aqueous solution of said cross-linking agent Ca⁺⁺ is prepared by dissolving CaCl₂ in water at a concentration in a range of 1.5% to 15% (g/100ml).

14. The method according to Claim 12, wherein the aqueous solution of said cross-linking agent Fe⁺⁺⁺ is prepared by dissolving FeCl₃·6H₂O in water at a concentration in a range of 2%to 15% (g/100ml).

15. The method according to Claim 12, wherein the aqueous solution of said cross-linking agent Al⁺⁺⁺ is prepared by dissolving AlCl₃·6H₂O in water at a concentration in a range of 1 %to 15% (g/100ml).

## Patentansprüche

1. Eine postoperative Adhesionsbarriere, umfassend:
Carboxymethylchitosan;
Carboxymethylcellulose; und
einen Vernetzer aus Metallionen.

2. Die postoperative Adhessionsbarriere gemäß Anspruch 1, worin der Substitutionsgrad des Carboxymethylteils des Carboxymethylchitosans 0,6 bis 1,2 ist.

3. Die postoperative Adhessionsbarriere gemäß Anspruch 1, worin der Substitutionsgrad des Carboxymethylteils der Carboxymethylcellulose 0,4 bis 1,2 ist.

4. Die postoperative Ädhessionsbarriere gemäß Anspruch 1, worin das Gewichtsverhältnis von Carboxymethylchitosan und Carboxymethylcellulose von 1:0,05 bis 1:2 ist.

5. Die postoperative Adhessionsbarriere gemäß Anspruch 1, worin der Vernetzer ausgewählt ist aus der Gruppe, bestehend aus Ca⁺⁺, Fe⁺⁺⁺, einer Mischung aus Ca⁺⁺ und Fe⁺⁺⁺, und einer Mischung aus Ca⁺⁺ und Al⁺⁺⁺.

6. Die postoperative Adhessionsbarriere gemäß den Ansprüchen 1 oder 5, worin die Metallionen aus CaCl₂, FeCl₃·6H₂O und AlCl₃·6H₂O stammen.

7. Die postoperative Adhessionsbarriere gemäß Anspruch 1 oder 5, worin das lonenäquivalentverhältnis von Ca⁺⁺ zu Fe⁺⁺⁺ der Mischung aus Ca⁺⁺ und Fe⁺⁺⁺ von 1:10 bis 1:0 ist.

8. Die postoperative Adhessionsbarriere gemäß Anspruch 5, worin das lonenäquivalentverhältnis von Ca⁺⁺ zu Al⁺⁺⁺ der Mischung aus Ca⁺⁺ und Al⁺⁺⁺ von 1:1 1 bis 1:0,05 ist.

9. Ein Verfahren zum Herstellen einer postoperativen Adhessionsbarriere, umfassend die folgenden Schritte:
(1) Herstellen einer ersten wässrigen Polymerlösung aus Carboxymethylchitosan und einer zweiten wässrigen Polymerlösung aus Carboxymethylcellulose;
(2) Herstellen einer wässrigen Lösung eines Metallvemetzers;
(3) Aufbringen einer Mischung der ersten wässrigen Polymerlösung und der zweiten wässrigen Polymerlösung auf die Oberfläche einer Platte, Trocknen der aufgebrachten wässrigen Polymerlösung auf der Platte, um einen Film auf der Platte zu erhalten;
(4) Tränken des getrockneten Films auf der Platte des Schritts (3) in der wässrigen Lösung des Schritts (2), um die Vernetzungsreaktion zu vervollständigen; und
(5) Trocknen des vernetzten Films auf der Platte des Schritts (4), anschließend Ablösen des Films von der Platte.

10. Das Verfahren gemäß Anspruch 9, worin die Konzentration des Carboxymethylchitosans in der ersten wässrigen Polymerlösung 1 bis 5% (g/100ml) ist.

11. Das Verfahren gemäß Anspruch 9, worin die Konzentration der Carboxymethylcellulose in der zweiten wässrigen Polymerlösung 2% bis 5% (g/100 ml) ist.

12. Das Verfahren gemäß Anspruch 9, worin der besagte Metallvernetzer ausgewählt ist aus der Gruppe, bestehend aus Ca⁺⁺, Fe⁺⁺⁺, einer Mischung aus Ca⁺⁺ und Fe⁺⁺⁺, und einer Mischung aus Ca⁺⁺ und Al⁺⁺⁺.

13. Das Verfahren gemäß Anspruch 12, worin die wässrige Lösung des besagten Vernetzers Ca⁺⁺ hergestellt wird durch Auflösen von CaCl₂ in Wasser in einem Konzentrationsbereich von 1,5% bis 15% (g/100 ml).

14. Das Verfahren gemäß Anspruch 12, worin die wässrige Lösung des besagten Vernetzers Fe⁺⁺⁺ hergestellt wird durch Auflösen von FeCl₃·6H₂O in Wasser in einem Konzentrationsbereich von 2 bis 15% (g/100 ml).

15. Das Verfahren gemäß Anspruch 12, worin die wässrige Lösung des besagten Vernetzers Al⁺⁺⁺ hergestellt wird durch Auflösen von AlCl₃·6H₂O in Wasser in einem Konzentrationsbereich von 1 bis 15% (g/100 ml).

## Revendications

1. Barrière contre l'adhérence post-chirurgicale, comprenant :
du carboxyméthylchitosane ;
de la carboxyméthylcellulose ; et
un agent de réticulation constitué d'ions métalliques.

2. Barrière contre l'adhérence post-chirurgicale selon la revendication 1, dans laquelle le degré de substitution du fragment carboxyméthyle du carboxyméthylchitosane est de 0,6 à 1,2.

3. Barrière contre l'adhérence post-chirurgicale selon la revendication 1, dans laquelle le degré de substitution du fragment carboxyméthyle de la carboxyméthylcellulose est de 0,4 à 1,2.

4. Barrière contre l'adhérence post-chirurgicale selon la revendication 1, dans laquelle le rapport en poids du carboxyméthylchitosane et de la carboxyméthylcellulose est de 1 : 0,05 à 1 : 2.

5. Barrière contre l'adhérence post-chirurgicale selon la revendication 1, dans laquelle l'agent de réticulation est choisi dans le groupe constitué par Ca⁺⁺, Fe⁺⁺⁺, un mélange de Ca⁺⁺ et de Fe⁺⁺⁺, et un mélange de Ca⁺⁺ et d'Al⁺⁺⁺.

6. Barrière contre l'adhérence post-chirurgicale selon la revendication 1 ou 5, dans laquelle les ions métalliques proviennent de CaCl₂, de FeCl₃•6H₂O et d'AlCl₃•6H₂O, respectivement.

7. Barrière contre l'adhérence post-chirurgicale selon la revendication 1 ou 5, dans laquelle le rapport en équivalent ion de Ca⁺⁺ sur Fe⁺⁺⁺ du mélange de Ca⁺⁺ et de Fe⁺⁺⁺ est de 1 : 10 à 1 : 0.

8. Barrière contre l'adhérence post-chirurgicale selon la revendication 5, dans laquelle le rapport en équivalent ion de Ca⁺⁺ sur Al⁺⁺⁺ du mélange de Ca⁺⁺ et d'Al⁺⁺⁺ est de 1 : 1 à 1 : 0,05.

9. Procédé de préparation d'une barrière contre l'adhérence post-chirurgicale, comprenant les étapes consistant à :
(1) préparer, respectivement, une première solution aqueuse de polymère de carboxyméthylchitosane et une seconde solution aqueuse de polymère de carboxyméthylcellulose ;
(2) préparer une solution aqueuse d'un agent de réticulation métallique ;
(3) appliquer un mélange de la première solution aqueuse de polymère et de la seconde solution aqueuse de polymère sur la surface d'une plaque, sécher la solution aqueuse de polymère appliquée sur la plaque pour obtenir un film sur la plaque ;
(4) tremper le film séché sur la plaque de l'étape (3) dans la solution aqueuse de l'étape (2) pour réaliser une réaction de réticulation ; et
(5) sécher le film réticulé sur la plaque de l'étape (4), puis détacher le film de la plaque.

10. Procédé selon la revendication 9, dans lequel la concentration du carboxyméthylchitosane dans la première solution aqueuse de polymère est de 1% à 5% (g/100 ml).

11. Procédé selon la revendication 9, dans lequel la concentration de la carboxyméthylcellulose dans la seconde solution aqueuse de polymère est de 2% à 5% (g/100 ml).

12. Procédé selon la revendication 9, dans lequel ledit agent de réticulation métallique est choisi dans le groupe constitué par Ca⁺⁺, Fe⁺⁺⁺, un mélange de Ca⁺⁺ et de Fe⁺⁺⁺, et un mélange de Ca⁺⁺ et d'Al⁺⁺⁺.

13. Procédé selon la revendication 12, dans lequel la solution aqueuse dudit agent de réticulation Ca⁺⁺ est préparée en dissolvant du CaCl₂ dans de l'eau à une concentration dans la plage de 1,5% à 15% (g/100 ml).

14. Procédé selon la revendication 12, dans lequel la solution aqueuse dudit agent de réticulation Fe⁺⁺⁺ est préparée en dissolvant du FeCl₃·6H₂O dans de l'eau à une concentration dans la plage de 2% à 15% (g/100 ml).

15. Procédé selon la revendication 12, dans lequel la solution aqueuse dudit agent de réticulation Al⁺⁺⁺ est préparée en dissolvant de l'AlCl₃•6H₂O dans de l'eau à une concentration dans la plage de 1% à 15% (g/100 ml).
